# EUROPEAN PATENT APPLICATION

(11) **EP 4 663 221 A1**
(43) Date of publication of application: **17.12.2025**
(21) Application number: 24305940.9
(22) Date of filing: 14.06.2024
(51) Int. Cl.: A61M 5/32

(54) **HOUSING DEVICE FOR RECEIVING AN INJECTION DEVICE, AND INJECTOR INCLUDING SAID HOUSING DEVICE AND INJECTION DEVICE**

(71) Applicant: Becton Dickinson France, 38800 Le Pont-de-Claix (FR)
(72) Inventor: FIARD, Michael, 38320 Eybens (FR); BESSON, Nicolas, 38650 Treffort (FR); CARREL, Franck, 38220 Saint Jean de Vaulx (FR)
(74) Representative: Germain Maureau

(57) **Abstract**

This housing device (1) includes: a bottom body (3) extending a longitudinal axis A, the bottom body (3) being configured for receiving an injection device (10) having an injection needle (102), a casing (110), a medical container (101) arranged within the casing (110), and a needle guard (112) axially movable with respect to the casing (110). The housing device also includes: a top body (2), axially movable with respect to the bottom body (3) between an initial position and an injection end position distally located with respect to the initial position, the top body (2) including a proximal end (201) and a plunger rod (22) configured for pushing a stopper (107) of the injection device (10) in order to expel a medical product contained within the medical container (101); releasable attachment features (71, 73) for allowing releasable attachment and detachment of the top body (2) and the bottom body (3); and a needle cover (4), axially movable with respect to the bottom body (3) between an initial position and a retracted position proximally located with respect to the initial position.

## Description

The present invention relates to a reusable housing for accommodating an injection device, and an injector including the reusable housing and the injection device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

It is known from document US9248242 an anti-needle stick safety device for an injection device. The user has to place his/her fingers on a finger flange of a needle guard and to push against a plunger head of a plunger rod in order to perform injection. This action requires some dexterity to be performed well. In particular, it is difficult to perform injection at the right injection depth.

There is therefore a need for a device that alleviates some or all of the aforementioned drawbacks of the prior art. Specifically, there is a need for a reusable device that is easier to grasp and to use, and that allows for controlling the injection depth provides an improved sustainability.

In this context, an aspect of the invention is a housing device including:
a bottom body extending a longitudinal axis A, the bottom body being configured for receiving an injection device having an injection needle, a casing, a medical container arranged within the casing, and a needle guard axially movable with respect to the casing,
a top body, axially movable with respect to the bottom body between an initial position and an injection end position distally located with respect to the initial position, the top body including a proximal end and a plunger rod configured for pushing a stopper of the injection device in order to expel a medical product contained within the medical container,
releasable attachment features for allowing releasable attachment and detachment of the top body and the bottom body, and
a needle cover, axially movable with respect to the bottom body between an initial position and a retracted position proximally located with respect to the initial position.

The housing device of the invention is thus reusable, and permits easier grasping for the user, due to its bottom body and top body encasing the injection device. The housing device of the invention also allows for a properly controlled injection depth due to this better grasp and the movement of the top body together with the plunger rod with respect to the bottom body.

The device of the invention may further include some or all of the features listed below.

In an embodiment, the housing device includes a holder for axially maintaining the injection device against the bottom body.

This helps control the sticking depth. The holder may be configured to exert a distal force on the injection device. The holder may include a resilient member for exerting a continuous distal push on the injection device.

In an embodiment, the housing device includes blocking features configured for axially blocking the top body with respect to the bottom body in the initial position until the injection needle of the injection device reaches a predetermined injection depth.

In an embodiment, the blocking features include a radially deformable leg, a radial stop for radially blocking the radially deformable leg in a blocking position, an inner surface for radially inwardly abutting against the injection device, and a release opening for allowing movement of the radially deformable leg from the blocking position to a release position when the needle cover reaches the retracted position.

In an embodiment, the needle cover includes a retainer for axially locking the needle cover to the bottom body when the needle cover reaches the retracted position.

The retainer prevents the needle cover from moving back in the distal direction under the force of the return spring.

In an embodiment, the proximal end of the top body includes a through-opening, and the housing device includes an indicating tab configured for engaging the through-opening when the top body reaches the injection end position in order to provide a user with a tactile and/or visual indication that injection is completed.

The indicating tab may be arranged on the bottom body or the needle cover. The indicating tab may extend along the longitudinal axis A.

In an embodiment, the proximal end of the top body includes a recess configured for receiving a user's thumb.

In an embodiment, the plunger rod is fixed to the top body.

More specifically, the top body includes a closed proximal end and the plunger rod is fixed to the closed proximal end of the top body. The plunger rod extends along the central longitudinal axis A.

In an embodiment, the releasable attachment features include a second snap-fit member connected to the top body or the bottom body by two pivoting arms, such that the second snap-fit member can be rotated around the pivoting arms between an engagement position and a release position.

The pivoting arms may extend at the same axial location, but may be inclined with respect to each other. In the engagement position, the second snap-fit member engages a first snap-fit member arranged on, respectively, the bottom body or the top body, such that the bottom body and the top body are axially blocked and thus attached to each other. In the release position, the second snap-fit member is away from the first snap-fit member to allow detachment of the bottom body and the top body.

In an embodiment, the housing device includes a cap removably attached to the bottom body, the cap including a remover for removing a needle shield of the injection device when the cap is being detached from the bottom body, and a disposal opening for allowing evacuation of the needle shield captured by the remover.

In an embodiment, the housing device includes a noise attenuator arranged at a proximal stop of the bottom body or at a distal stop of the needle cover for attenuating noise generated by the abutment of this distal stop against this proximal stop when the needle cover reaches the initial position.

Another aspect of the invention is an injector including the aforementioned housing device and an injection device arranged within the housing device.

The injection device may include an injection needle, a casing, a medical container arranged within the casing, a stopper and a needle guard axially movable with respect to the casing.

The invention and the advantages arising therefrom will clearly emerge from the detailed description that is given below with reference to the appended drawings as follows :
- Figure 1A is a perspective view of a housing device according to an embodiment of the invention,
- Figure 1B is an exploded view of a housing device and an injector according to an embodiment of the invention,
- Figure 1C is an exploded view of an injection device device according to an embodiment of the invention,
- Figures 2A, 2B are perspective views of a top body of a housing device according to an embodiment of the invention,
- Figure 2C is a cross section view of a top body of a housing device according to an embodiment of the invention,
- Figure 2D is a perspective view of a holder of a housing device according to an embodiment of the invention,
- Figure 2E is a cross-section view of a top body of a housing device according to another embodiment of the invention,
- Figures 3A, 3B are perspective views of a bottom body of a housing device according to an embodiment of the invention,
- Figures 4A, 4B are perspective views of a needle cover of a housing device according to an embodiment of the invention,
- Figures 5A, 5B are perspective views of a cap of a housing device according to an embodiment of the invention,
- Figures 6A, 6B are cross section views of a housing device and injector according to an embodiment of the invention, in two longitudinal planes orthogonal to each other,
- Figure 7A is a cross-section view of a housing device according to an embodiment of the invention, the needle cover being in the retracted position,
- Figure 7B is a detail of Figure 7A,
- Figure 8 is a cross-section view of a housing device according to an embodiment of the invention, the top body being in the injection end position,
- Figures 9A, 9B are cross-section views of a housing device according to an embodiment of the invention, when the top body moves back to its initial position,
- Figures 10A-10G and 11 illustrate steps of use of a housing device and injector according to an embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

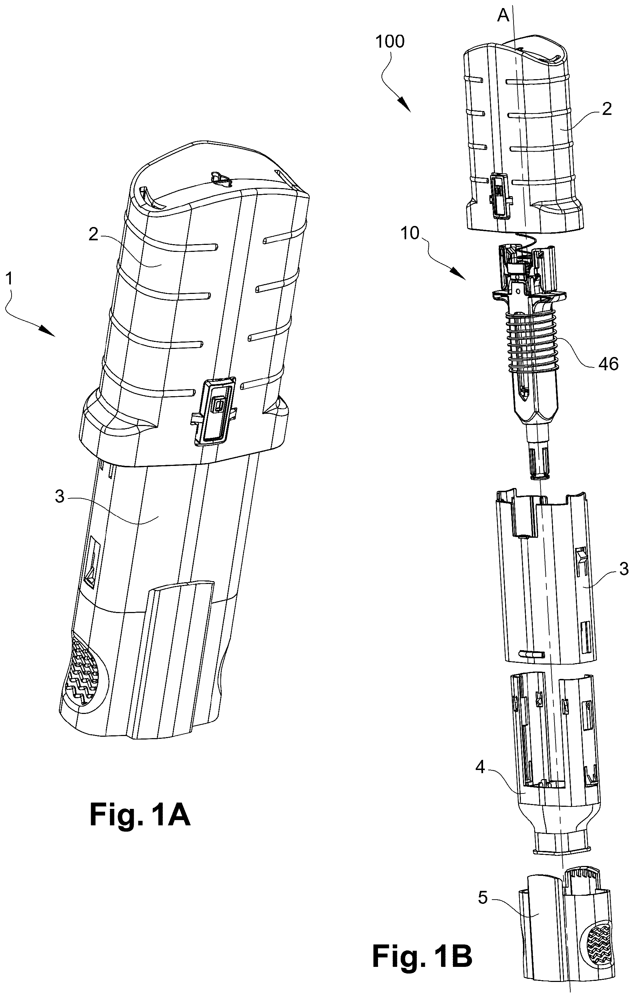

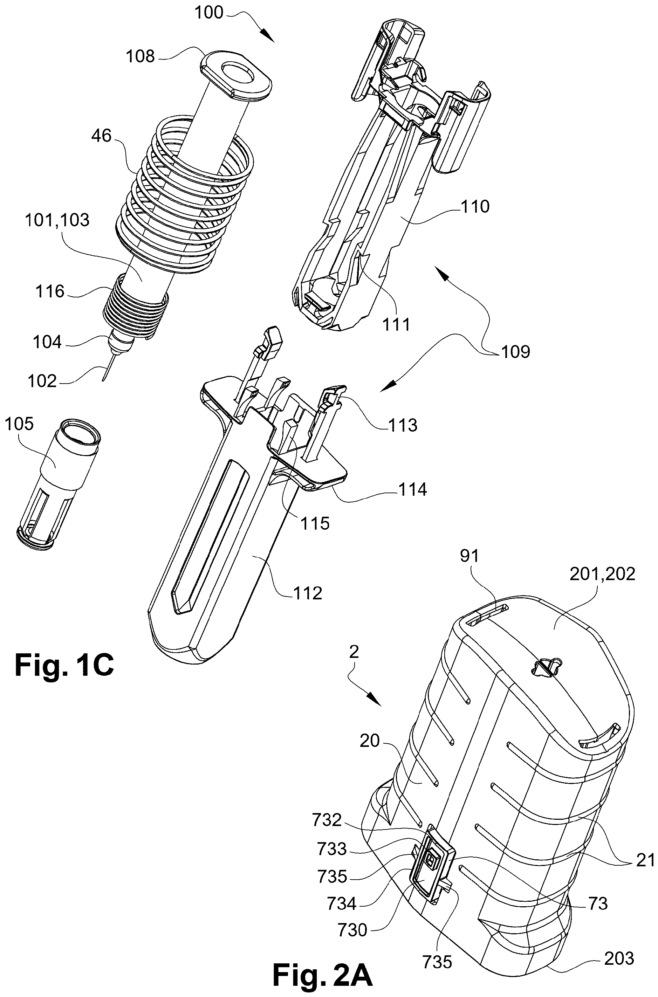

With reference to Figures 1A-1B is shown a reusable housing device 1 according to an embodiment of the invention. The housing 1 of the invention is configured for receiving an injection device 10 and for being grasped by a user in order to perform an injection operation. The housing 1 of the invention is also configured for allowing insertion of an injection needle 102 of the injection device 10 at a predetermined injection depth.

As illustrated in Figures 1B-2, the injection device 10 may include a medical container 101, such as a prefilled syringe, and an anti-needle stick safety device 109 receiving the medical container 101. The anti-needle stick safety device 109 may be similar to the one described in the document US9248242.

The medical container 101 may include a tubular barrel 103 defining a reservoir configured for containing a medical product. The tubular barrel 103 may include an elongated distal tip 104 defining an axial passageway for passage of the medical product from the reservoir and an injection needle 102 attached to this distal tip. Prior to use of the injection device 10, the injection needle 102 is sealed and protected by a removable needle shield 105 removably attached to the distal tip 104. A stopper 107 is arranged within the barrel 103 for pushing fluid outside the barrel 103 via the distal tip and the injection needle 102. At its proximal end, the barrel 103 may include a radial flange 108 which may serve to hold the medical container 101 at a fixed axial position within a casing 110 of an anti-needle stick safety device 109. The barrel 103 may include a glass or a plastic material.

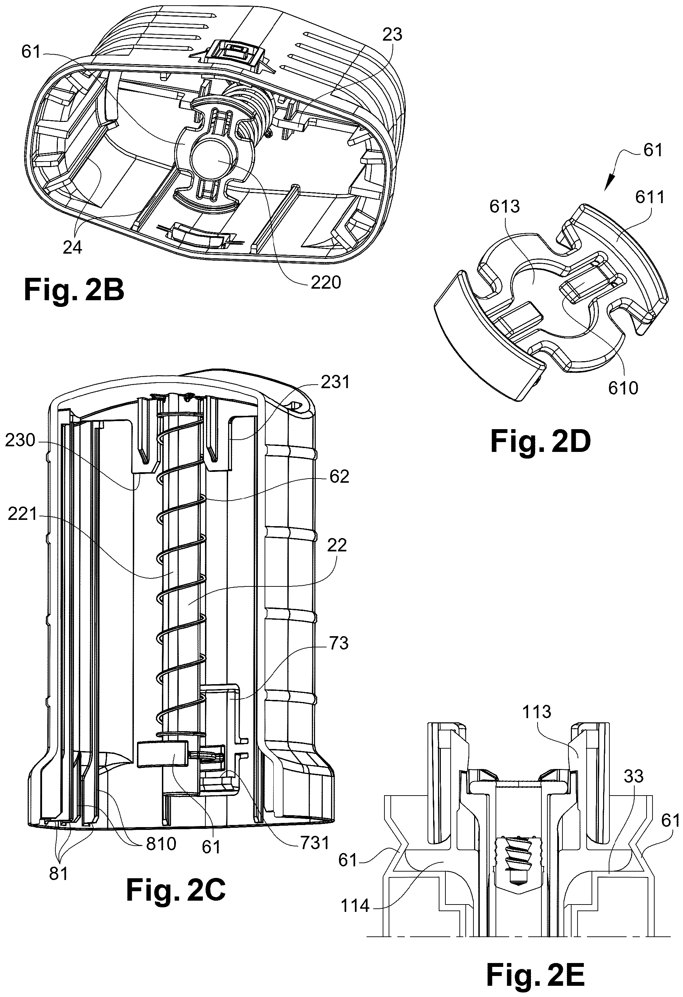

Still with reference to Figure 2, the anti-needle stick safety device 109 may include a casing 110 for accommodating the medical container 101 and a needle guard 112 for protecting against needle stick injuries. The casing 110 may be arranged within the needle guard 112. The needle guard 112 is axially movable with respect to the housing between an initial (retracted) position, and a safety (extended) position, distally located with respect to the initial position. The safety device 109 further includes a safety spring 116 for moving the needle guard 112 in the distal direction, and one or more trigger fingers 113. The trigger fingers 113 are arranged on the needle guard 112 for abutting against the casing 110 to maintain the needle guard 112 in the initial position. The trigger fingers 113 are radially outwardly deflectable to release the needle guard 112 from the casing 110 such that the needle guard 112 can move to the safety position. The needle guard 112 may include a finger flange 114. The safety device 109 may further include a locker for locking the needle guard 112 in the safety position. The locker may include a distal stop 111 and a tab 115 for axially abutting against the distal stop in the safety position. The tab may be arranged on the needle guard 112 whereas the distal stop may be arranged on the casing 110.

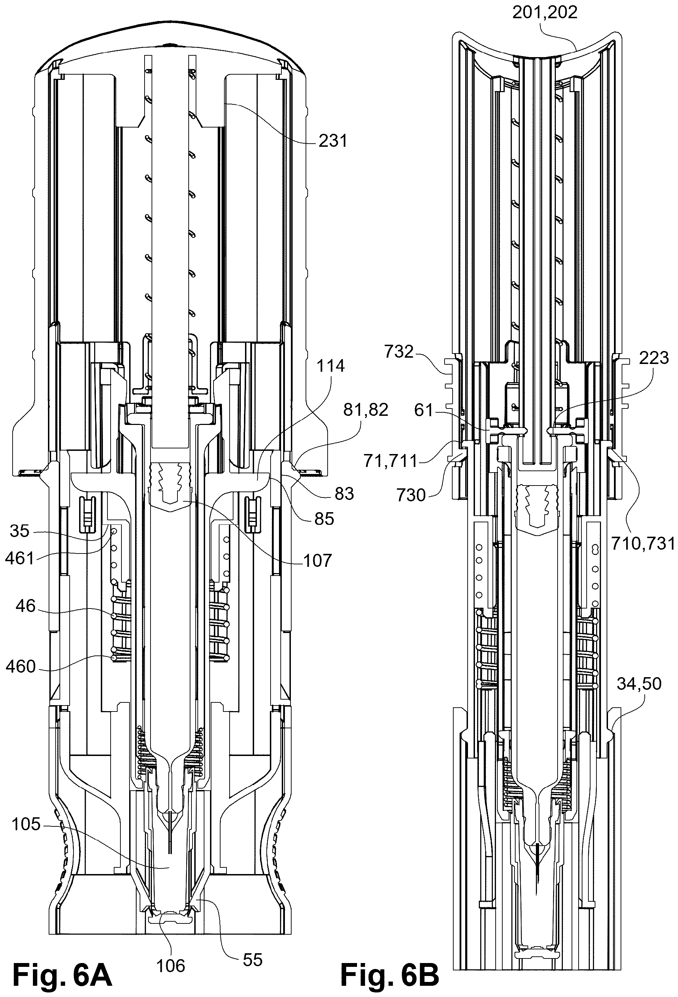

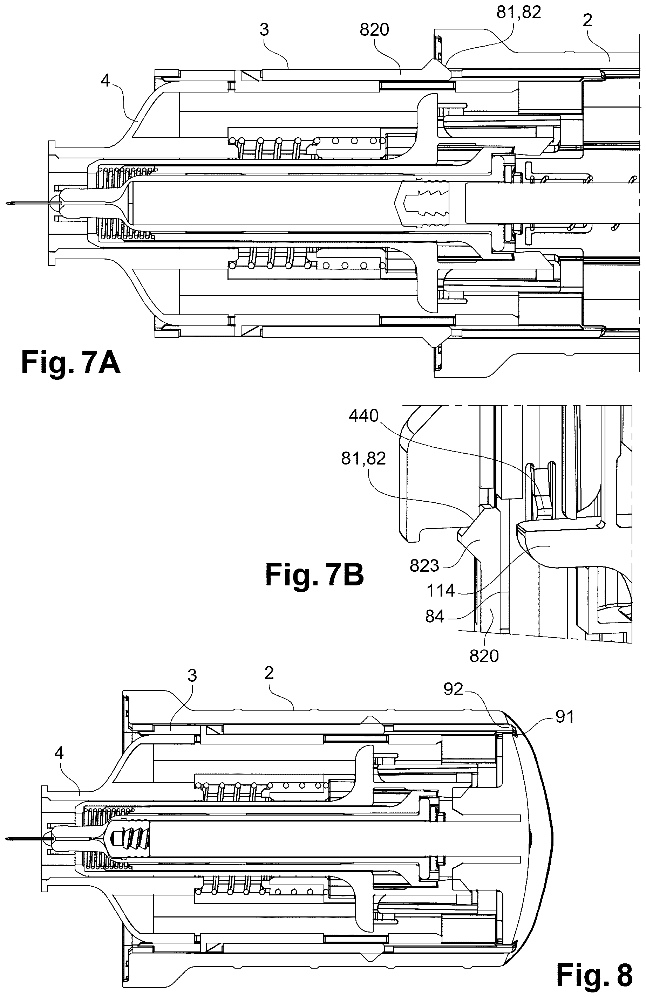

With reference to Figures 2A-2C, the housing 1 of the invention extends along a longitudinal axis A and may include a top body 2, a bottom body 3, a needle cover 4, and a removable cap 5. The needle cover 4 may be arranged within the bottom body 3, and may be axially movable with respect to the bottom body 3 between an initial position, Figure 6A, in which the needle cover 4 may be configured for hiding the injection needle 102 of the medical container 101, and a retracted position, Figure 7A, proximally located with respect to the initial position, in which the needle cover 4 allows the user for performing injection (and more specifically allows the user for pushing the stopper 107 in order to expel the medical product contained within the barrel 103). Movement of the needle cover 4 from the initial position to the retracted position is caused by the user pressing a distal end 40 of the needle cover 4 against an injection site. A return spring 46 is arranged between the bottom body 3 and the needle cover 4 to force the needle cover 4 towards the initial position.

The top body 2 includes a tubular lateral wall 20 defining an inner cavity for receiving the bottom body 3, a proximal end 201 which may be closed, and a distal end 203 which defines a distal opening for receiving the bottom body 3. The closed proximal end 201 may have a concave shape, thereby forming a recess 202 for accommodating the user's thumb. This helps the user push the top body 2 in the distal direction to perform injection. The lateral wall 20 of the top body 2 may further have a somewhat oval or ovoid cross-section shape. This provides a more comfortable grip to the end user. The top body 2 may include gripping protrusions 21 such as ribs arranged on an outer surface of the lateral wall for easing grip of the housing device 1 by the user. The top body 2 is configured for allowing a full hand grip, which makes it more ergonomic.

As illustrated in Figure 2C, the top body 2 may include a plunger rod 22, centrally axially extending within the inner cavity of the top body 2 and distally protruding from the proximal end of the top body 2. The plunger rod 22 includes a proximal end attached to the top body 2, and a distal end 220, Figure 2B, configured for pushing the stopper 107 within the barrel 103 in order to expel the medical product. The plunger rod 22 may be fixed to the top body 2, thereby moving together with the top body 2. In the illustrated embodiment, the top body 2 and the plunger rod 22 may be made of a single piece.

The top body 2 further includes one or more trigger members 23, such as ribs, which may be arranged within the inner cavity of the top body 2, for engaging the trigger fingers 113 of the injection device 10 in order to move the trigger fingers 113 to their release position. The top body 2 may include two diametrically opposite trigger members 23, which axially extend from the closed proximal end 201 of the top body 2, and which may be arranged on both sides of the plunger rod 22. The trigger members 23 have a proximal end secured to the top body 2, an opposite distal end 230, and an outer side which may define a side engagement surface 231 for radially abutting against the trigger fingers 113 and maintaining the trigger fingers 113 in the release position. The trigger members 23 may be fixed to the top body 2, thereby moving together with the top body 2. In the illustrated embodiment, the top body 2 and the plunger rod 22 may be made of a single piece.

Still with reference to Figure 2B, the top body 2 may include guiding ribs 24, axially extending within the inner cavity, along an inner surface of the lateral wall 20, for guiding axial movement of the top body 2 relative to the bottom body 3. The top body 2 indeed is axially movable with respect to the bottom body 3 (and so is consequently the plunger rod 22 with respect to the injection device 10) between an initial position, Figure 6A, in which the bottom body 3 extends outside the top body 2 such that the plunger rod 22 has not started yet to push the stopper 107 (the plunger rod 22 may even be away from the stopper 107), and an injection end position, Figure 8, in which the bottom body 3 is retracted within the top body 2 and in which the plunger rod 22 presses the stopper 107 against a distal end of the reservoir delimited by the barrel 103. Distal movement of the top body 2 from the initial position to the injection end position is caused by the user pushing the top body 2 in the distal direction while the needle cover 4 abuts against the injection site. Proximal movement of the top body 2 back to the initial position is caused by a safety spring 116 of the injection device 10 and/or a holding spring 62 of the housing device 1, as will be described in further details below.

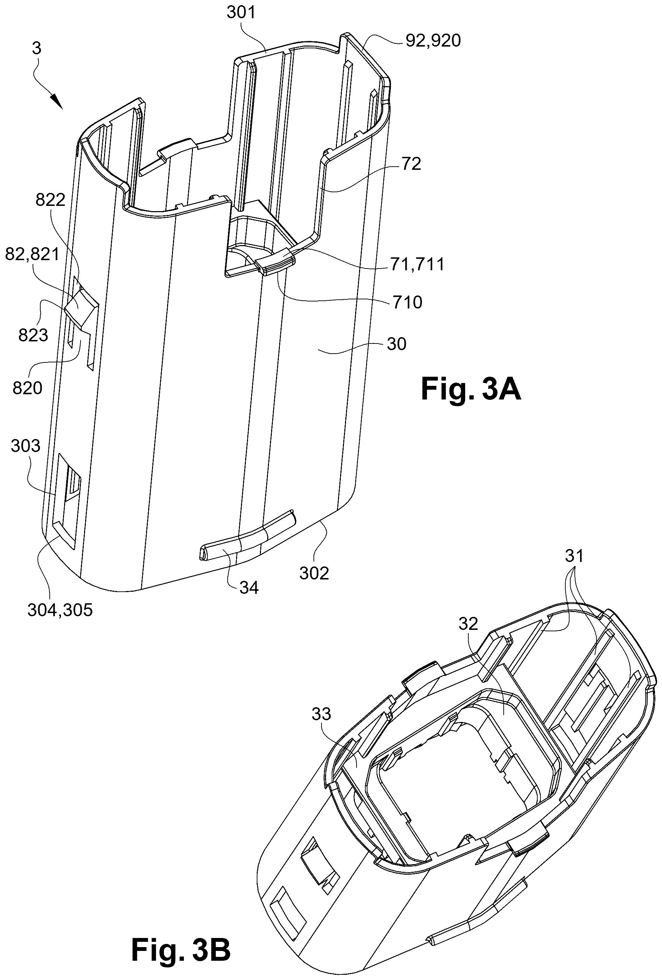

With reference to Figures 3A-3B, the bottom body 3 includes a sidewall 30 defining an inner cavity for receiving the needle cover 4, an opened proximal end 301 and an opened distal end 302 for allowing passage of the needle cover 4. The bottom body 3 further includes guiding ribs 31, axially extending along an inner surface of the sidewall 30, for guiding axial movement of the needle cover 4 with respect to the bottom body 3, and a proximal stop 304, which may be arranged at a distal end of a sliding window 303 for axially abutting against a distal stop 450 of the needle cover 4. A noise attenuator 305, for instance made by overmolding, may be arranged at the proximal stop 304 of the bottom body 3 or at the distal stop 450 of the needle cover 4 to avoid noise when the needle cover 4 hits the proximal stop 304 (when the housing device 1 is reset in order to be re-used). The sliding window 303 extends through the sidewall 30 of the bottom body 3 and is configured to receive a guiding protrusion 41 of the needle cover 4. The bottom body 3 also includes a recess 32 which may be shaped to receive the medical container 101 of the injection device 10 and a proximal shoulder 33 for axially abutting against a distal side of the finger flange 114 of the injection device 10. Therefore the injection device 10 is distally and radially blocked within the bottom body 3. The bottom body 3 may further include, on an outer surface of its sidewall 30, a proximal seat 34 for axially abutting against a distal abutment 52 of the cap 5, in order to keep the cap 5 attached to the bottom body 3.

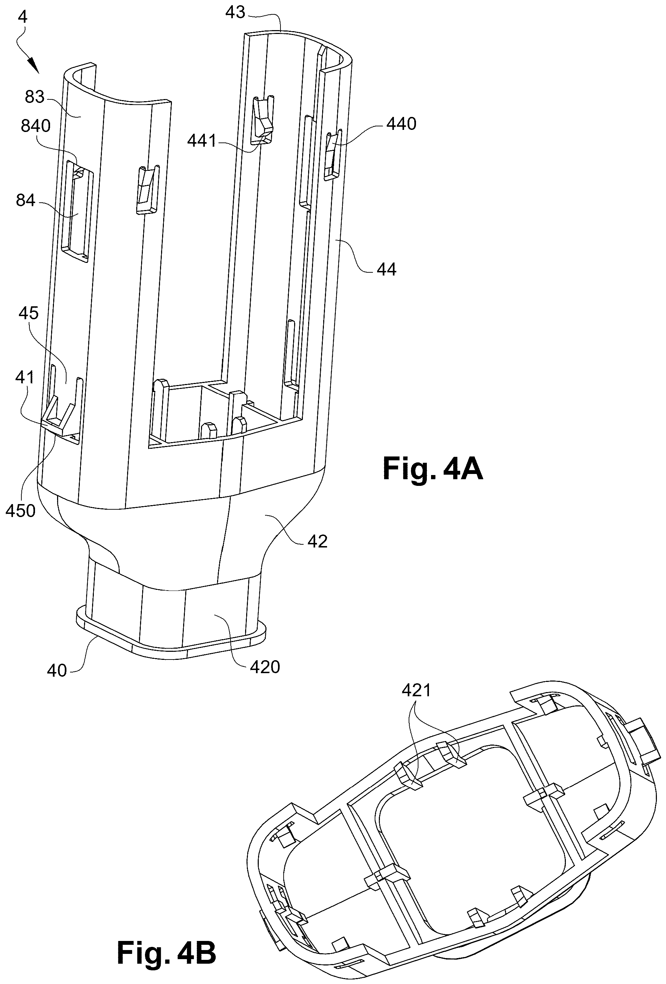

As illustrated in Figures 4A-4B, the needle cover 4 may include a lateral wall 42 defining a sleeve portion 420 for surrounding the injection needle 102, a distal end 40 configured for being pressed against the injection site, a proximal end 43, and one or more proximal legs 44 axially extending from the lateral wall 42 to the proximal end 43 of the needle cover 4. The needle cover 4 may include guiding ribs 421 axially extending within the sleeve portion 420 for guiding axial movement of the cap 5 when the cap 5 is being detached from the bottom body 3. The needle cover 4 may further include one or more, for instance two diametrically opposite, flexible tabs 45 having an outward guiding protrusion engaged within the sliding window 303 of the bottom body 3. The tab 45, at its distal side, delimits the distal stop 450 of the needle cover 4. In its initial position, the distal stop 450 of the needle cover 4 axially abuts against the proximal stop 304 of the bottom body 3.

The needle cover 4 further includes one or more, for instance four, retainers 440 which may be arranged on the proximal legs 44, near the proximal end 43 of the needle cover 4, for retaining the needle cover 4 in the retracted position. The retainers 440 may be in the form of resiliently deformable snap-fit members 441 having a distal abutment 441 configured for axially abutting against a proximal side of the finger flange 114 of the injection device 10, Figure 7B. Retaining the needle cover 4 in the retracted position avoids interference between the needle cover 4 and a blocking feature 820 of the bottom body 3 such that this blocking feature 820 can move back to its initial position during disassembly of the housing device 1.

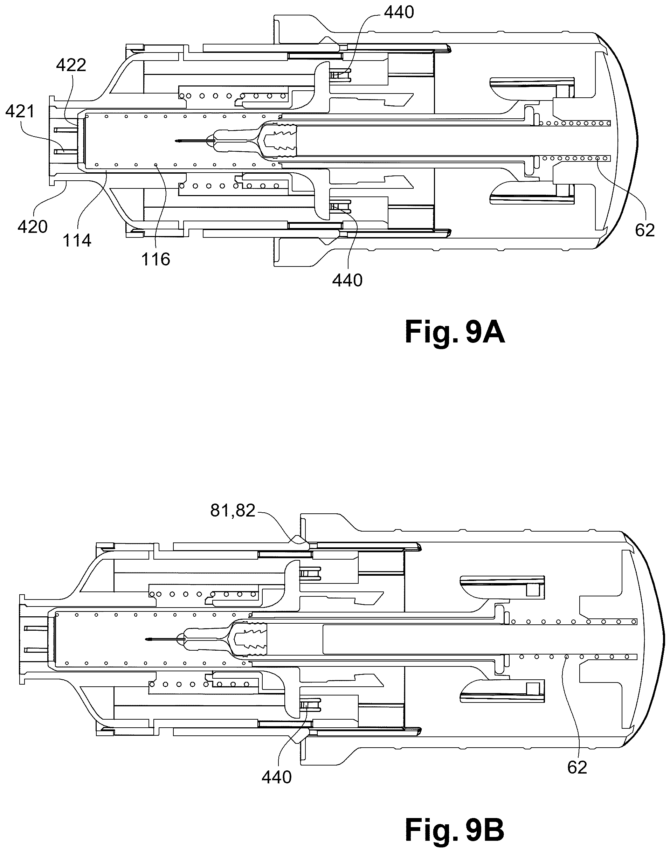

The needle cover 4 may further include a proximal abutment 422 for axially abutting against a distal end of the needle guard 112 of the injection device 10. The proximal abutment 422 may be arranged on the axial ribs 421 extending along an inner wall of the sleeve portion 420 of the needle cover 4, Figure 9A. This abutment allows the safety spring 116 of the injection device 10 for pushing the top body 2 back in the proximal direction when the safety spring 116 is being deployed after injection. The safety spring 116 pushes the casing 110 and thus the medical container 101 in the proximal direction and, due to the abutment between the medical container 101 and the plunger rod 22 via the stopper 107, pushes the plunger rod 22 (which is fixed to the top body 2), in the proximal direction. In an alternative embodiment, the needle cover 4 is devoid of such a proximal abutment 422 and directly presses against the injection site to push the top body 2 in the proximal direction.

With reference to Figure 6A, the return spring 46 may be a coil spring having a distal end 460 leaning against a proximal seat of the needle cover 4 and a proximal end 461 leaning against a distal shoulder 35 of the bottom body 3. Therefore, the return spring 46 tends to push the needle cover 4 away from the bottom body 3.

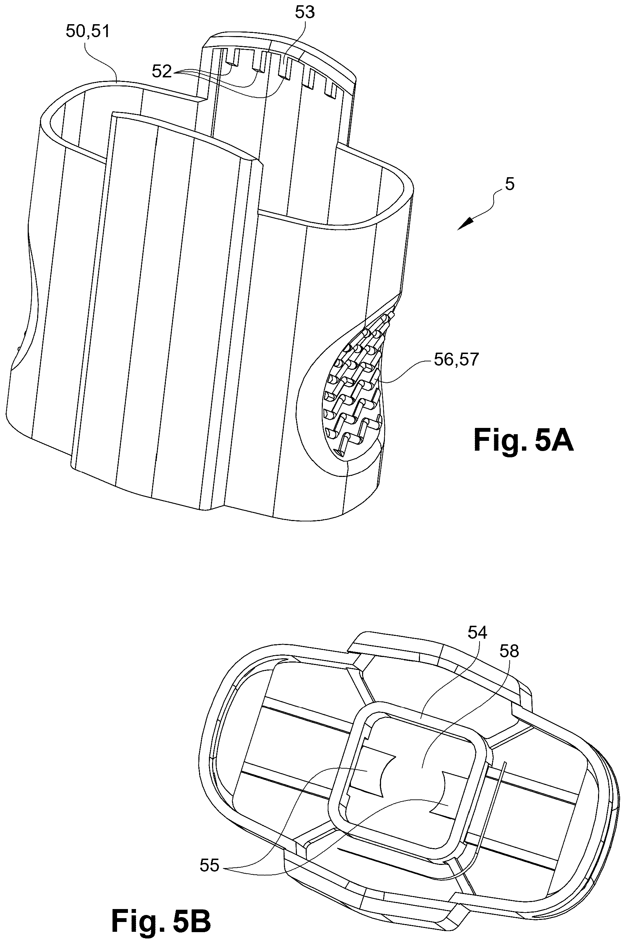

As illustrated in Figures 5A-5B, the removable cap 5 may include some or all of a proximal abutment 50, arranged at a proximal end 51 of the cap 5, for axially abutting against the distal end 302 of the bottom body 3, a distal abutment 52, arranged at a distal end of one or more ribs 53, for axially abutting against the proximal seat 34 of the bottom body 3, a guiding element 54, in the form of an axial inner sleeve defining an inner cavity, for guiding withdrawal of the cap 5 along the longitudinal axis A with respect to the needle cover 4, a remover 55, in the form of flexible tongues arranged in the inner cavity of the cap 5, for removing the needle shield 105 when the cap 5 is detached from the bottom body 3, one or more gripping members 56 in the form of protrusions arranged on an outer surface of the cap 5, in a recessed portion 57 thereof, for easing grip of the cap 5 by the user, and a disposal opening 58 for allowing removal of the needle shield 105 from the cap 5 such that the needle shield 105 can be disposed and the cap 5 can be re-used. The disposal opening 58 may be arranged at a distal end of the cap 5 and opens into the inner cavity for allowing withdrawal of the needle shield 105 captured within the inner cavity by moving the needle shield 105 in the distal direction with respect to the cap 5. To that end, the remover 55, which prevents proximal movement of the needle shield 105 relative to the cap 5, is configured for allowing distal movement of the needle shield 105 relative to the cap 5 such that the needle shield 105 can be inserted in the cap 5 and later removed from the cap 5. The remover 55 may be configured for clamping a distal end 106 of the needle shield 105.

With reference to Figures 2D and 2E, the housing device 1 may further include a holding unit 61, 62 configured for axially blocking the injection device 10 in the bottom body 3. In the embodiment illustrated in Figure 2D, the holding unit 61, 62 is arranged in the top body 2 and prevents proximal movement of the injection device 10. The holding unit includes a holder 61 for distally pressing against a radial flange 108 of the injection device 10, and a holding spring 62 for moving the holder 61 in the distal direction. The holding spring 62 forms a resilient member for distally pushing the injection device 10 in the distal direction. The holder 61 includes a resilient guiding element 610, such as one or more flexible and inwardly radially extending arms, which may extend within corresponding axial slots 221 provided through the plunger rod 22. A distal end of these slots forms a proximal stop such that the holder 61 remains assembled to the plunger rod 22 when the top body 2 is separated from the bottom body 3. The holding spring 62 may be a coil spring extending between the closed proximal end of the top body 2 and a proximal side of the holder 61. In the illustrated embodiment, the holding spring 62 is arranged around the plunger rod 22. The holder 61 may also include one or more radial shoulders 611 for radially abutting against the radial flange 108 and thus securing the radial flange 108 of the injection device 10 with respect to the radial direction. The holder 61 defines a central opening 613 which may be sized to slide along the plunger rod 22 ; the diameter of the central opening 613 may be slightly greater or equal to the diameter of the plunger rod 22.

In an alternative embodiment illustrated in Figure 2E, the holding unit is arranged on the bottom body 3. The holder 61 may be a resiliently deformable tab arranged on the lateral wall of the bottom body 3 for abutting against a proximal side of the finger flange 114 of the injection device 10 in order to axially press the finger flange 114 against the proximal shoulder 33 of the bottom body 3, such that the finger flange 114 is captured between the proximal shoulder 33 and the holder 61 of the bottom body 3. The holder 61 may be radially ouwtardly deformable to allow for passage of the finger flange 114 over the holder 61 in the distal direction (when inserting the injection device 10 into the bottom body 3) and in the proximal direction (when removing the used injection device 10 from the bottom body 3). The tab forms a resilient member for distally pushing the injection device 10 in the distal direction.

With reference to Figure 6B, and 2A-3A, the housing device 1 includes releasable attachment features for releasably attaching the bottom body 3 to the top body 2. The releasable attachment features may include snap-fit members : a first snap-fit member 71, which may be arranged on the bottom body 3, and a second snap-fit member 73, which may be arranged on the top body 2. The first snap-fit member 71 includes a distal stop 710 defined at a distal side of a tangential rib 711 which outwardly protrudes from an outer surface of the sidewall 30 of the bottom body 3. The first snap-fit member 71 may be formed at a distal end of a notch 72 whose proximal end opens at the proximal end 301 of the bottom body 3 and which is configured to receive the second snap-fit member 73.

The second snap-fit member 73 may be in the form of a flip-up arm including, on an inner surface of a distal portion 730, a proximal stop 731 for axially abutting against the distal stop 710 of the first snap-fit member 71, and, on an outer surface of a proximal portion 732, a pressing side surface 733 for allowing the user to radially inwardly press the proximal portion 732 of the second snap-fit member 73 such that the distal portion 730 move radially outwardly away from the first snap-fit member 71. This allows for disassembly of the top body 2 and the bottom body 3. The second snap-fit member 73 is thus movable between an engagement position in which its proximal stop 731 axially abuts against the distal stop 710 of the first snap-fit member 71, and a release position in which the proximal stop 731 and the distal stop 710 are radially shifted away from each other. The second snap-fit member 73 may be arranged in a window 734 through the lateral wall 20 of the top body 2 and may be connected to the lateral wall 20 of the top body 2 by two pivoting arms 735 extending in a tangential direction and allowing for rotation of the second snap-fit member 73 with respect to the lateral wall of the top body 2. The two pivoting arms 735 separate the distal portion 730 and the proximal portion 732 of the second snap-fit member 73.

In an alternative embodiment, the first snap-fit member 71 may be arranged on the top body 2 and the second snap-fit member 73 on the bottom body 3.

The housing device 1 here includes two-diametrically opposite first snap-fit members 71 and two diametrically opposite second snap-fit members 73, although embodiments are not limited thereto.

With reference to Figure 6A, and 3A-4A, the housing device 1 may include one or more, for instance two diametrically opposite, blocking features 81, 82, 820 for preventing distal movement of the the top body 2 with respect to the bottom body 3 before complete injection needle 102 insertion. The blocking features include a distal stop 81, which may be arranged on the top body 2, and a proximal stop 82, which may be arranged on the bottom body 3. The distal stop 81 may be delimited by the distal end 203 of the top body 2 and/or at a distal end of an inner axial rib 810 of the top body 2. The proximal stop 82 may be arranged on a resiliently deformable leg 820 of the bottom body 3. The resiliently deformable leg 820 may proximally extend from a distal end of a window 822 provided through the sidewall 30 of the bottom body 3. The resilient leg 820 has a distal end fixed to the bottom body 3 and an opposite proximal end 821 which may have an outward radial protrusion 823 whose proximal side may delimit the proximal stop 82 of the blocking features. The proximal side and the distal side of this protrusion 823 may be inclined with respect to the longitudinal axis to ease inward deformation of the resilient leg 820 from a blocking position to a release position, radially inwardly located with respect to the blocking position.

To prevent inward movement of the resilient leg 820 from the blocking position to the reslease position, the housing device 1 includes a radial stop 83 which may be arranged on the needle cover 4. The radial stop 83 may be an outer surface of the proximal legs 820 of the needle cover 4. The radial stop 83 may extend between a proximal end 821 of the proximal legs of the needle cover 4 and a proximal end 840 of a release opening 84 arranged through the needle cover 4. Radially opposite the outer surface that defines the radial stop 83, the needle cover 4 may include an inner surface 85 configured for radially inwardly abutting against the finger flange 114 of the injection device 10, Figure 6A. Thus, the finger flange 114 helps rigidity the needle cover 4 to prevent inward deformation of the resilient leg 820 of the bottom body 3.

The release opening 84 is provided on the proximal legs 44 of the needle cover 4 for allowing inward radial deformation of the resilient legs 820 forming the blocking features. That is, as long as the needle cover 4 is away from the retracted position, the radial stop 83 radially faces the resilient leg 820 of the blocking features such that this resilient leg 820 cannot move to the release position and is therefore constrained to stay in the blocking position. The release opening 84 is arranged to radially face the resilient leg 820 when the needle cover 4 is in the retracted position. Thus, the top body 2 can move over the protrusion 823 of the resilient leg 820 only when the needle cover 4 is in the retracted position, i.e. when the predetermined injection depth is reached by the injection needle 102.

The blocking features 810, 820, the radial stop 83 and the release opening 84 therefore act as sequencing means configured for putting in sequence insertion of the injection needle and injection of the medical product, i.e. for allowing injection of the medical product only after insertion of the injection needle 102 at a predetermined injection depth.

With reference to Figure 8, and 2A-3A, the housing device 1 may include an indicator 91, 92 for indicating the end of injection. This indicator may be configured to provide a tactile and/or visible feedback to the user. For instance, the indicator may include a through-opening 91, which may be arranged on the top body 2, and an indicating tab 92, which may be arranged on the bottom body 3, for engaging the through-opening 91. The through-opening 91 may extend through the closed proximal end 201 of the top body 2, and more specifically through the recess 202 accommodating the user's thumb. Besides, the indicating tab 92 may be configured to extend through and proximally protrude from the though-opening 91, such that the user can feel the indicating tab 92 pressing against his/her thumb when injection is completed. The indicating tab 92 and the closed proximal end 201 of the top body 2 may also have different colors. As visible in Figure 3A, the indicating tab 92 may be in the form of an axial projection 920 proximally extending from the proximal end 301 of the bottom body 3. The indicator may include one or more, such as two diametrically opposite, indicating tabs 92 and through-openings 91.

Another aspect of the invention is an injector 100 including the above-described injection device 10 and an injection device 10 received received within the housing device 1.

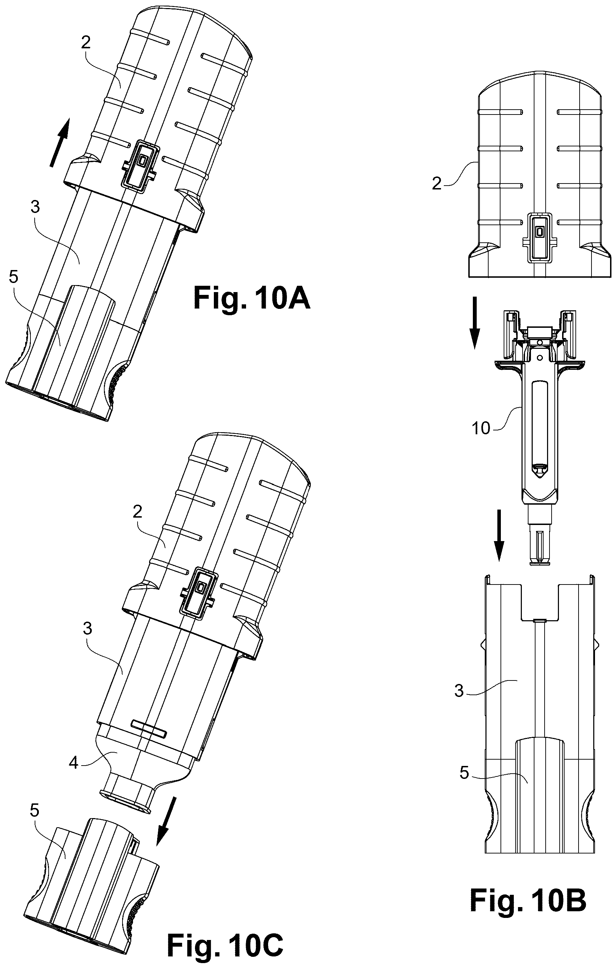

Figures 10A-10B illustrate how the injection de vice 10 is place within the housing device 1. First, the user radially presses the snap-fit members 72 of the top body 2 and pulls the top body 2 in the proximal direction so that the top body 2 and the bottom body 3 can get detached, Figure 10A. Then, the user inserts the injection device 10 within the bottom body 3, Figure 10B, until the injection device 10 axially abuts against the proximal shoulder 33 of the bottom body 3. The needle shield 105 engages the cap 5, and gets gripped by the remover 55, Figure 6A. The user then reassembles the top body 2 and the bottom body 3 by pushing the top body 2 against the bottom body 3 such that the first snap-fit member 71 and the second snap-fit member 73 engage each other. The top body 2 axially abuts against the bottom body 3 by means of the blocking features 810, 820. The holder 61 of the top body 2 engages the injection device 10 and axially maintain the injection device 10 against the bottom body 3.

Figures 10C to 10G illustrate use of the injector 100 of the invention.

First the user removes the cap 5 from the bottom body 3, Figure 10C. This entails removal of the needle shield 105 from the injection device 10. The needle shield 105 may stay captured within the cap 5.

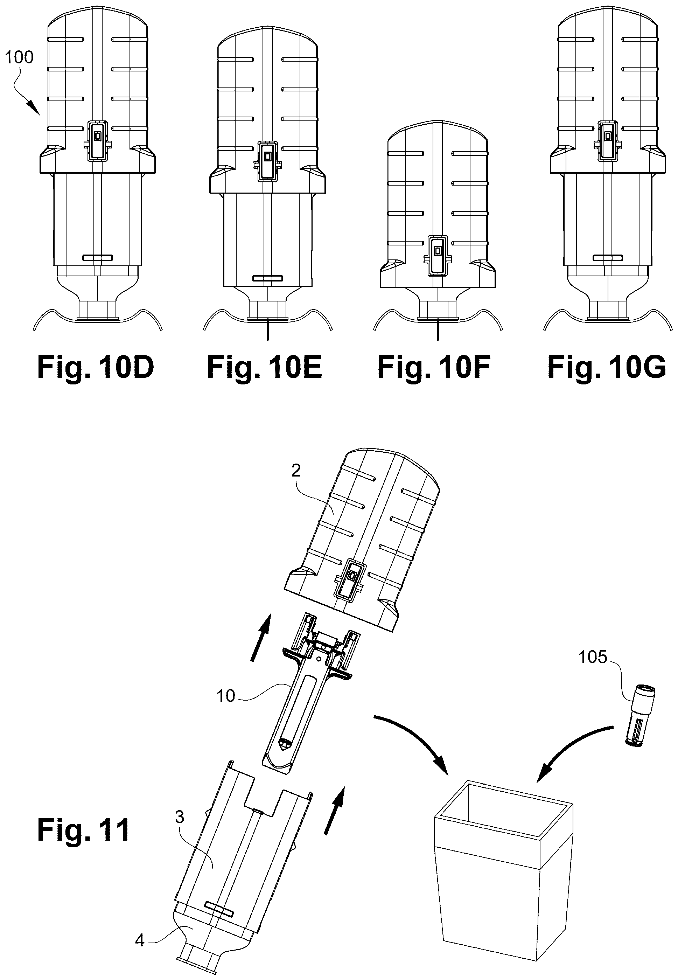

The user then presses the distal end 40 of the needle cover 4 against the injection site, Figure 10D. The top body 2 cannot move along the bottom body 3 due to the blocking features 810, 820. Thus, the needle cover 4 slides towards its retracted position. The injection needle 102 accordingly penetrates into the injection site, Figure 10E.

When the injection needle 102 reaches the predetermined injection depth, the needle cover 4 reaches the retracted position. The retainers 440 of the needle cover 4 engage the finger flange 114 of the injection device 10, such that the needle cover 4 will stay in the retracted position despite the action of the return spring 46. Besides, the resilient leg 820 now faces the release opening 84 of the needle cover 4 and is free to move to the release position. The distal force exerted by the user on the top body 2 causes the top body 2 to deflect the resilient leg 820 to its release position. The top body 2 passes over the protrusion and moves in the distal direction with respect to the bottom body 3, Figure 10F.

This causes the plunger rod 22 to push the stopper 107 in the distal direction inside the barrel 103, thereby expelling the medical product contained within the barrel 103. When injection is complete, that is when the stopper 107 reaches the distal end of the barrel 103, the indicating tabs 92 of the bottom body 3 engage the through-openings 91 of the top body 2, thus informing the user that injection is done. The user can then remove the injector 100 from the injection site.

Besides, at the end of the injection, the trigger members 23 of the top body 2 engage and deflect the trigger fingers 113 of the injection device 10. As a result, the casing 110 of the injection device 10 is now free to move with respect to the needle guard 112 of the injection device 10. Since the needle guard 112 is blocked in the distal direction by the needle cover 4 or by the injection site, the expansion of the safety spring 116 causes the casing 110 of the injection device 10 to be moved in the proximal direction, Figure 10G. Due to the axial abutment between the casing 110, the barrel 103, the stopper 107 and the plunger rod 22, the top body 2 is also moved proximally. Initially, it is the safety spring 116 which moves the top body 2 in the proximal direction, Figure 9A, because the safety spring 116 may be stronger than the holding spring 62. But at some point, the holding spring 62 takes over and finishes moving the top body 2 in the proximal direction, Figure 9B, until the top body 2 returns to ist initial position, passing back over the protrusion 823 of the bottom body 3.

After that, the user can open the housing device 1 in order to dispose the used injection device 10. To that end, the user presses the snap-fit members 72 of the top body 2 and pulls the top body 2 in the proximal direction so that the top body 2 and the bottom body 3 can get detached, Figure 11. Then, the used injection device 10 is pulled from the bottom body 3. This action deflects the retainers 440 which release the needle cover 4 from the finger flange 114. The needle cover 4 thus moves back to the initial position under the force of the return spring 46. As said earlier, the housing device 1 can include a noise attenuator 305 for limiting the noise caused by the distal stop of the needle cover 4 hitting the proximal stop of the bottom body 3. The used medical container 101 is discarded in an appropriate container.

The needle shield 105 captured by the cap 5 can be pushed in the distal direction to get detached from the remover 55 and exit the cap 5 via the disposal opening 58. The needle shield 105 can be discarded in an appropriate container while the cap 5 can be reattached to the bottom body 3. The user can put the top body 2 back onto the bottom body 3, and the cap 5 onto the bottom body 3, so that the housing device 1 can be re-used instead of being thrown away.

It is readily understandable from the above description that the housing device 1 of the invention is advantageously reusable. This improves sustainability. Besides, the housing device 1 can be easily handled by the user and the injection can be performed at the proper injection depth. It is to be understood that the present invention is not limited to the embodiments described above and illustrated in the drawings; rather, the skilled person will recognize that many changes and modifications may be made within the scope of the appended claims.

## Claims

1. Housing device (1) including:
a bottom body (3) extending a longitudinal axis A, the bottom body (3) being configured for receiving an injection device (10) having an injection needle (102), a casing (110), a medical container (101) arranged within the casing (110), and a needle guard (112) axially movable with respect to the casing (110),
a top body (2), axially movable with respect to the bottom body (3) between an initial position and an injection end position distally located with respect to the initial position, the top body (2) including a proximal end (201) and a plunger rod (22) configured for pushing a stopper (107) of the injection device (10) in order to expel a medical product contained within the medical container (101),
releasable attachment features (71, 73) for allowing releasable attachment and detachment of the top body (2) and the bottom body (3), and
a needle cover (4), axially movable with respect to the bottom body (3) between an initial position and a retracted position proximally located with respect to the initial position.

2. Housing device (1) according to the preceding claim, wherein the housing device (1) includes a holder (61) for axially maintaining the injection device (10) against the bottom body (3).

3. Housing device (1) according to any one of the preceding claims, wherein the housing device (1) includes blocking features (810, 820) configured for axially blocking the top body (2) with respect to the bottom body (3) in the initial position until the injection needle (102) of the injection device (10) reaches a predetermined injection depth.

4. Housing device (1) according to the preceding claim, wherein the blocking features (810, 820) include a radially deformable leg (820), a radial stop (83) for radially blocking the radially deformable leg (820) in a blocking position, an inner surface (85) for radially inwardly abutting against the injection device (10), and a release opening (84) for allowing movement of the radially deformable leg (820) from the blocking position to a release position when the needle cover (4) reaches the retracted position.

5. Housing device (1) according to any one of the preceding claims, wherein, the needle cover (4) includes a retainer (440) for axially locking the needle cover (4) to the bottom body (3) when the needle cover (4) reaches the retracted position.

6. Housing device (1) according to any one of the preceding claims, wherein the proximal end (201) of the top body (2) includes a through-opening (91), and the housing device (1) includes an indicating tab (92) configured for engaging the through-opening (91) when the top body (2) reaches the injection end position in order to provide a user with a tactile and/or visual indication that injection is completed.

7. Housing device (1) according to any one of the preceding claims, wherein the proximal end (201) of the top body (2) includes a recess (202) configured for receiving a user's thumb.

8. Housing device (1) according to any one of the preceding claims, wherein the plunger rod (22) is fixed to the top body (2).

9. Housing device (1) according to any one of the preceding claims, wherein the releasable attachment features include a second snap-fit member (73) connected to the top body (2) or the bottom body (3) by two pivoting arms (735), such that the second snap-fit member (73) can be rotated around the pivoting arms (735) between an engagement position and a release position.

10. Housing device (1) according to any one of the preceding claims, wherein the housing device (1) includes a cap (5) removably attached to the bottom body (3), the cap (5) including a remover (55) for removing a needle shield (105) of the injection device (10) when the cap (5) is being detached from the bottom body (3), and a disposal opening (58) for allowing evacuation of the needle shield (105) captured by the remover (55).

11. Housing device (1) according to any one of the preceding claims, wherein the housing device (1) includes a noise attenuator (305) arranged at a proximal stop (304) of the bottom body (3) or at a distal stop (450) of the needle cover (4) for attenuating noise generated by the abutment of this distal stop (450) against this proximal stop (304) when the needle cover (4) reaches the initial position.

12. Injector (100) including a housing device (1) according to any one of the preceding claims, and an injection device (10) arranged within the housing device (1).
